# EUROPEAN PATENT APPLICATION

(11) **EP 3 072 436 A1**
(43) Date of publication of application: **28.09.2016**
(21) Application number: 15776644.5
(22) Date of filing: 07.04.2015
(51) Int. Cl.: A61B 1/00, G02B 23/24

(54) **ENDOSCOPE**

(30) Priority: 08.04.2014 JP 2014079765
(71) Applicant: Olympus Corporation, Shibuya-ku Tokyo 151-0072 (JP)
(72) Inventor: FUJITANI, Kiwamu, Hachioji-shi Tokyo 192-8507 (JP)
(74) Representative: Gunzelmann, Rainer
(86) International application number: PCT/JP2015/060855
(87) International publication number: WO 2015/156284

(57) **Abstract**

An endoscope includes: a long insertion portion sequentially provided with first and second bending portions and a flexible tube portion, from a distal end side; first and second tubular portions including first and second notch portions provided inside of the first and second bending portions, respectively; and a third tubular portion including a third notch portion provided inside of the flexible tube portion, wherein the first to third tubular portions are formed in one tubular member, the first notch portion is formed by first notches bendable in at least two directions, the second tubular portion includes second notches formed in a direction different from the two directions, and distal end portions of coil pipes into which pulling wires are inserted to be able to freely advance and retract are fixed to inside of the second tubular portion.

## Description

### Technical Field

The present invention relates to an endoscope including an insertion portion provided with a bending portion.

### Background Art

In recent years, an endoscope including an insertion portion provided with a bending portion has been widely used in a medical field and the like. To perform an endoscopic examination, diagnosis or the like by inserting an insertion portion into a lumen, such as a bronchus, including a three-dimensionally intricate conduit structure in which a conduit branches into a plurality of conduits in the middle of the conduit, an endoscope provided with a first bending portion as an actively bent bending portion as well as a passively bent second bending portion is used in some cases.

In a conventional example of such an endoscope in Japanese Patent Application Laid-Open Publication No. 2012-528651, the endoscope includes an insertion portion sequentially provided with a first bending portion, a second bending portion, and a flexible tube portion from a distal end side, and the first bending portion is bent and driven in two directions by operation of a lever provided on a handle on a proximal end side of the insertion portion through a set of wires.

In this Japanese Patent Application Laid-Open Publication No. 2012-528651, a single tube for forming the first bending portion, the second bending portion, and the flexible tube portion is provided with a plurality of grooves (notches) in a longitudinal direction of the tube, and patterns of the plurality of grooves (such as distance between adjacent grooves) are formed such that the patterns are different between the first bending portion and the second bending portion.

In the conventional example, the single tube is used to form the first bending portion and the second bending portion. Therefore, generation of a difference in level at a joint when the first bending portion and the second bending portion are formed by different members can be eliminated, and the structure allows easily reducing the diameter of the insertion portion. However, there is a drawback that a bending function of the second bending portion cannot be sufficiently secured.

More specifically, if the first bending portion has a function of bending in two directions, a user can combine operation of rotating the insertion portion about an axis direction to bring a direction of bending into line with a direction of the curved conduit. However, when the second bending portion has a bending function for the same two directions as in the first bending portion, excellent insertion cannot be secured in inserting the second bending portion into the intricately curved conduit.

In the conventional example, when the wires are pulled to bend the first bending portion, and the first bending portion is bent in a direction of the pulled wires, the second bend portion follows the bending direction of the first bending portion and is also bent.

When the distal end side of the insertion portion is inserted into the three-dimensionally and intricately curved conduit, the first bending portion is bent in a curving direction of the curved conduit that is an insertion target in front of the first bending portion, for example. However, it is desirable that the second bending portion has a function of passively bending along a curve of the conduit on a back side of the curved conduit that is the insertion target. When a curving direction of the conduit on the back side is different from the curving direction of the insertion target in front, the conventional example has a drawback that excellent insertion cannot be realized.

The present invention has been made in view of the foregoing circumstances, and an object of the present invention is to provide an endoscope that can secure excellent insertion into a three-dimensionally and intricately curved conduit.

### Disclosure of Invention

### Means for Solving the Problem

An aspect of the present invention provides an endoscope including: a long insertion portion inserted into a subject and sequentially provided with a first bending portion, a second bending portion, and a flexible tube portion, from a distal end side; a first tubular portion provided inside of the first bending portion, wherein a first notch portion defining a bending direction is provided in a longitudinal direction, pulling wires are fixed to a distal end portion of the insertion portion, and the pulling wires are disposed inside; a second tubular portion provided inside of the second bending portion, wherein a second notch portion in a pattern different from a pattern of the first notch portion is provided in a longitudinal direction; and a third tubular portion provided inside of the flexible tube portion, wherein a third notch portion in a pattern different from the patterns of the first and second notch portions is provided in a longitudinal direction different from the direction in which the second notch portion is provided, wherein the first to third tubular portions are formed in one tubular member, the first notch portion is formed of first notches bendable at least in two directions, the second notch portion includes second notches formed in the two directions and third notches formed in a direction different from the two directions, and in the second tubular portion, distal end portions of coil pipes into which the pulling wires are inserted to be able to freely advance and retract are fixed to inside of the second tubular portion.

### Brief Description of the Drawings

Fig. 1 is a diagram showing an endoscope apparatus including an endoscope of a first embodiment of the present invention;
Fig. 2 is a cross-sectional view showing a structure of an insertion portion of the endoscope;
Fig. 3 is a side view showing a tubular member in Fig. 2;
Fig. 4 is a diagram showing a distal end surface of the insertion portion;
Fig. 5 is an enlarged view of an A-A' line cross section in Fig. 2;
Fig. 6 is an enlarged view of a B-B' line cross section in Fig. 3;
Fig. 7A is an explanatory view of action in the present embodiment;
Fig. 7B is an explanatory view of action in insertion into a side deeper than in Fig. 7A;
Fig. 7C is an explanatory view of action in insertion into a side deeper than in Fig. 7B;
Fig. 8 is a diagram showing a structure of notch portions provided in a tube body according to a first modification of the first embodiment of the present invention;
Fig. 9 is a diagram showing a structure of notch portions provided in the tube body according to a second modification of the first embodiment of the present invention;
Fig. 10 is a diagram showing a structure of notch portions provided in the tube body according to a third modification of the first embodiment of the present invention;
Fig. 11 is a diagram showing a structure of notch portions provided in the tube body according to a fourth modification of the first embodiment of the present invention;
Fig. 12 is a diagram showing a structure of notch portions provided in the tube body according to a fifth modification of the first embodiment of the present invention;
Fig. 13 is a diagram showing a structure of notch portions provided in the tube body according to a sixth modification of the first embodiment of the present invention;
Fig. 14 is a diagram showing a structure of notch portions provided in the tube body according to a seventh modification of the first embodiment of the present invention;
Fig. 15 is a diagram showing a structure of notch portions provided in the tube body according to an eighth modification of the first embodiment of the present invention;
Fig. 16 is a diagram showing a structure of notch portions provided in the tube body according to a ninth modification of the first embodiment of the present invention; and
Fig. 17 is a diagram showing a structure of notch portions provided in the tube body according to a tenth modification of the first embodiment of the present invention.

### Best Mode for Carrying Out the Invention

Hereinafter, an embodiment of the present invention will be described with reference to the drawings.

### (First Embodiment)

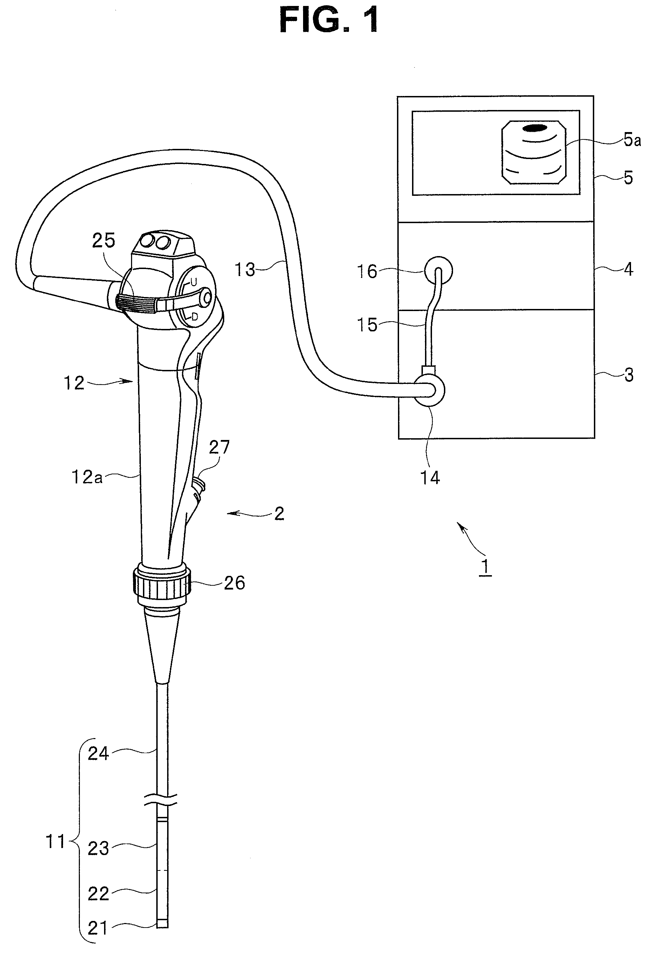

An endoscope apparatus 1 shown in Fig. 1 includes: an endoscope 2 of a first embodiment of the present invention; a light source apparatus 3 connected to a cable of the endoscope 2 and configured to supply illuminating light to the endoscope 2; a video processor 4 as a signal processing apparatus configured to drive an image pickup section of the endoscope 2 and apply signal processing to a signal picked up by the image pickup section; and a monitor 5 as a display apparatus configured to receive a video signal (image signal) generated by the video processor 4 and display, as an endoscopic image, an image of an object, such as a diseased part in a body cavity, picked up by the image pickup section.

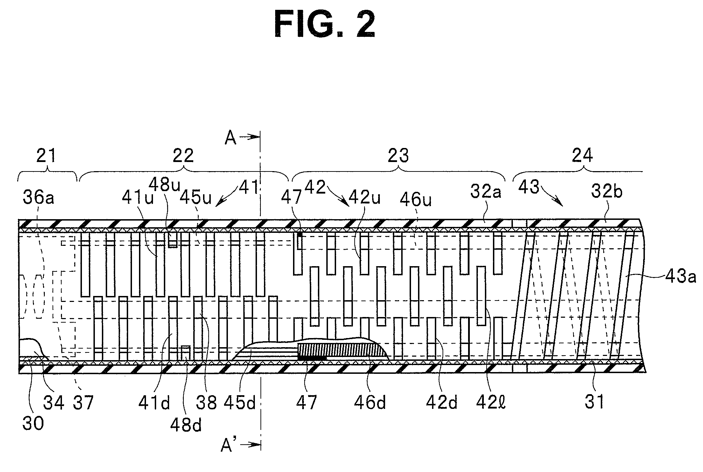

The endoscope 2 includes: an elongated and flexible insertion portion 11; an operation portion 12 provided with a grasping portion 12a grasped by a user, such as an operator, provided on a back end (proximal end) of the insertion portion 11; and a cable 13 extended from the operation portion 12. A light source connector 14 on an end portion of the cable 13 is detachably connected to the light source apparatus 3. An incident end portion of a light guide (see Fig. 2) inserted into the endoscope 2 reaches the light source connector 14, and the light source apparatus 3 supplies illuminating light to the incident end portion of the light guide.

A signal connector 16 on an end portion of an extension cable 15 extended from the light source connector 14 is detachably connected to the video processor 4.

The insertion portion 11 includes: a rigid distal end portion 21 provided on a distal end of the insertion portion 11; a first bending portion 22 provided on a proximal end of the distal end portion 21 and configured to actively bend; a second bending portion 23 provided on a proximal end of the first bending portion 22 and configured to passively bend; and a long flexible tube portion 24 extended from a proximal end of the second bending portion 23 to a front end of the operation portion 12.

In the present embodiment, the first bending portion 22 is configured to bend in two directions, upward and downward, for example. A bending operation knob 25 is provided at a position where fingers of a grasping hand in the grasping portion 12a can reach, and a user can perform operation of rotating (vertically moving) the bending operation knob 25 in the upward direction or the downward direction from a state of a neutral position shown in Fig. 1 to thereby bend the first bending portion 22 in the upward or downward direction. On a back end of the insertion portion 11 (flexible tube portion 24 of the insertion portion 11), a rotation operation portion 26 forming a rotation mechanism configured to rotate the insertion portion 11 relative to the operation portion 12 is provided to cover a front end of the operation portion 12 and a cap of a back end of the insertion portion 11.

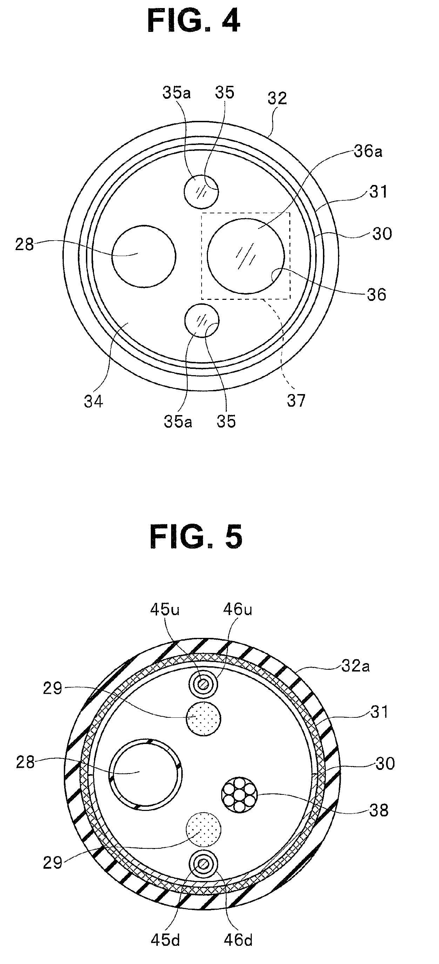

The rotation operation portion 26 is integrally fixed to the insertion portion 11, and the user can perform operation of rotating the rotation operation portion 26 in a circumferential direction to rotate the insertion portion 11 relative to the operation portion 12. A treatment instrument insertion port 27 for inserting a treatment instrument is provided at a circumference position of the operation portion 12 on a back end side of the rotation operation portion 26, and the treatment instrument insertion port 27 communicates with a treatment instrument channel 28 (see Figs. 4 and 5) provided in a longitudinal direction in the insertion portion 11. Note that a configuration of a rotation operation portion 41 disclosed in Japanese Patent Application Laid-Open Publication No. 2010-69108 can be utilized for the rotation operation portion 26.

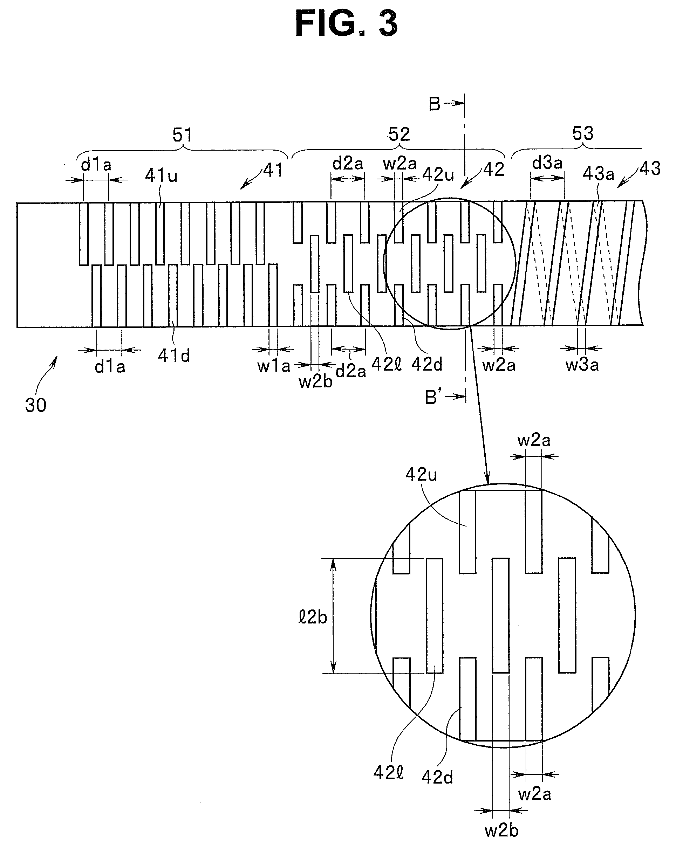

Fig. 2 shows a structure of a distal end side of the insertion portion 11, and Fig. 3 shows a side view of a tube body as a tubular member included in the insertion portion 11. Note that Fig. 2 shows a cross-sectional view of an elastic member and a resin member that are a mesh tube and an outer skin member on an outer side of the tube body, and part of the tube body is cut off to show a pulling wire and a coil pipe inside of the tube body.

As shown in Fig. 2, the insertion portion 11 includes: a tube body 30 as a tubular member; a mesh tube 31 provided on a circumference surface of the tube body 30; and a tubular outer skin member provided outside of the mesh tube 31.

In the present embodiment, the tube body 30 as a tubular member forming the insertion portion 11 is formed by a single cylindrical tube from the distal end portion 21 to the proximal end of the flexible tube portion 24. The tube body 30 is formed by a shape memory alloy, such as a nickel-titanium alloy (NiTi), with a high elastic deformation.

The mesh tube 31 is provided to cover the circumference surface of the tube body 30.

The outer skin member provided outside of the mesh tube 31 is formed by a rubber tube 32a as a tubular elastic member with elasticity outside of the distal end portion 21, the first bending portion 22, and the second bending portion 23 and is formed by a resin tube 32b as a tubular resin member with flexibility outside of the flexible tube portion 24. A proximal end of the rubber tube 32a and a front end of the resin tube 32b are connected by an adhesive or the like.

Note that although the rubber tube 32a is an outer skin member of the distal end portion 21 in the example shown in Fig. 2, the outside of the distal end portion 21 may be formed by, for example, a resin tube different from the rubber tube 32a.

A distal end member 34 in a columnar shape is, for example, fitted into a distal end part of the tube body 30 and bonded by an adhesive or the like to form the rigid distal end portion 21.

A front surface of the distal end member 34 serves as a distal end surface of the distal end portion 21, and on the distal end surface, a distal end opening of the treatment instrument channel 28 and an observation window 36 are provided adjacent to each other in, for example, a horizontal direction as shown in Fig. 4. On the distal end surface, respective illumination lenses 35a for emitting illuminating light are attached to, for example, two illumination windows 35 provided at positions in a vertical direction. Each of the illumination lenses 35a expands the illuminating light transmitted by a light guide 29 (see cross-sectional view of Fig. 5) inserted into the endoscope 2 including inside of the insertion portion 11 and emits the illuminating light to a front side of the distal end surface.

An objective lens 36a is attached to the observation window 36, and for example, a charge coupled device (abbreviated as CCD) 37 is arranged as an image pickup device at an image formation position of the objective lens 36a. The objective lens 36a forms an optical image of an object, such as a diseased part, illuminated by the illuminating light emitted from the illumination window 35 on an image pickup surface of the CCD 37, and (the objective lens 36a and) the CCD 37 forms an image pickup section configured to output an image pickup signal obtained by picking up an image of the object. The treatment instrument insertion port 27 shown in Fig. 1 communicates with a back end side of the treatment instrument channel 28 arranged in a longitudinal direction of the insertion portion 11 as shown in Fig. 5, and a distal end of the treatment instrument channel 28 opens in a round shape on the distal end surface as shown in Fig. 4. A distal end side of the treatment instrument inserted into the treatment instrument insertion port 27 can be caused to protrude from the distal end opening of the treatment instrument channel 28 to perform, for example, a treatment of collecting living tissue of a diseased part under observation by the endoscope 2. Note that the treatment instrument channel 28 is formed by a hollow tube as shown in Fig. 5.

A distal end of a signal cable 38 inserted into the endoscope 2 including the insertion portion 11 is connected to the CCD 37, and the other end of the signal cable 38 is connected to the video processor 4 through the extension cable 15 extended from the light source connector 14. The video processor 4 applies a CCD drive signal to the CCD 37 to apply signal processing to the image pickup signal picked up by the CCD 37 and outputs a video signal generated by the signal processing to the monitor 5. The monitor 5 displays the image picked up by the CCD 37 as an endoscopic image on an endoscopic image display area 5a on the display surface as shown for example in Fig. 1.

Note that an orientation of the image pickup surface of the CCD 37 fixed in the distal end portion 21 is set to coincide with a bending direction that is a standard in the first bending portion 22. An upward direction and a downward direction of the endoscopic image when the image picked up by the CCD 37 is displayed as an endoscopic image coincide with the upward direction and the downward direction of bending (upper bending direction and lower bending direction) of the first bending portion 22.

Therefore, for example, when a dark part exists on an upper side of the endoscopic image in the endoscopic image as shown in Fig. 1, and the distal end side of the insertion portion 11 is to be bent in a direction of the dark part, the user can operate the bending operation knob 25 to bend the first bending portion 22 in the upward direction.

In the present embodiment, as shown in Figs. 2 and 3, a first notch portion 41, a second notch portion 42, and a third notch portion 43 with notches in different patterns are formed in the longitudinal direction of a circular tube surface of the tube body 30, and a first tubular portion 51, a second tubular portion 52, and a third tubular portion 53 forming the first bending portion 22, the second bending portion 23, and the flexible tube portion 24 bendable toward the sides provided with the notches are formed (51, 52, and 53 are not shown in Fig. 2, and 22, 23, and 24 are not shown in Fig. 3).

In other words, the first notch portion 41, the second notch portion 42, and the third notch portion 43 in different patterns are provided to the tube body 30 part serving as inside parts of the first bending portion 22, the second bending portion 23, and the flexible tube portion 24, respectively, to form the first tubular portion 51, the second tubular portion 52, and the third tubular portion 53 to thereby constitute the first bending portion 22, the second bending portion 23, and the flexible tube portion 24, respectively.

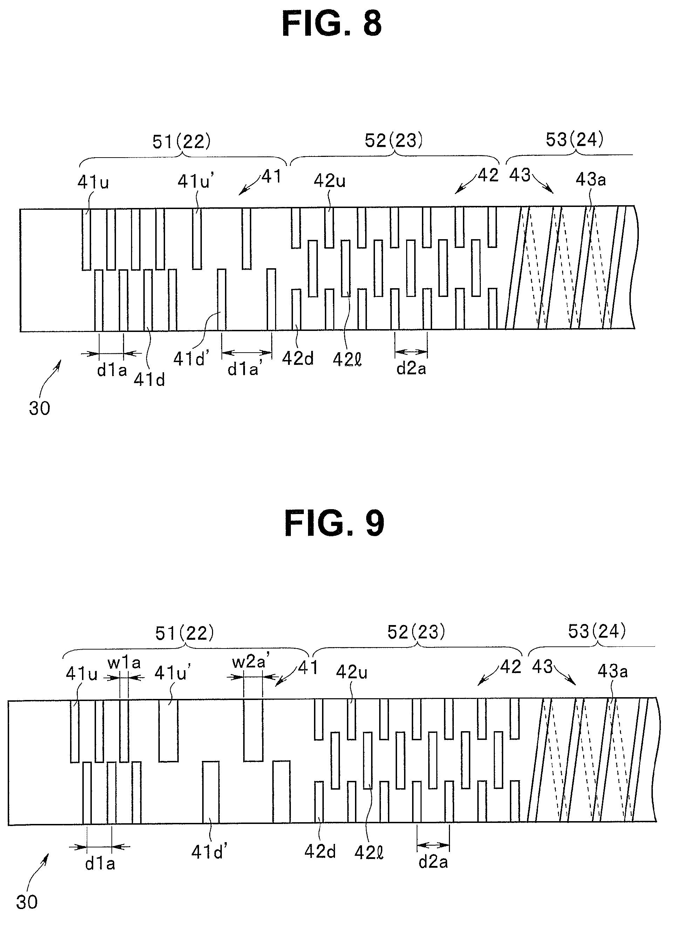

In the first tubular portion 51, first notches 41 u and 41 d forming the first notch portion 41 are formed in the longitudinal direction of the circular tube surface of the tube body 30, at intervals (pitch) of d1a, with a notch width w1a (in the longitudinal direction of the circular tube surface), and substantially symmetrically along corresponding positions in the upward and downward directions of bending, for example. Note that the vertical direction of the paper surface of Figs. 2 and 3 corresponds to the vertical direction of bending. The same applies to Figs. 8 to 17 in modifications described later.

In the present embodiment (and the modifications described later), the intervals (pitch) of the notches provided in the longitudinal direction of the tube body 30 are indicated by distances between notch center positions of the notches adjacent in the longitudinal direction.

The first notches 41u are formed with a length half the length of the circular tube surface in the circumferential direction as shown in Fig. 5 (upper semicircular part is cut off). Assuming that a length of the tube body 30 in the circumferential direction is L, a length 11u of the first notches 41u is L/2. Note that the length 11u of the first notches 41u is not limited to L/2, and the length 11u may be a larger value or a smaller value. The first notches 41u are provided horizontally symmetrically in the circumferential direction on left and right sides based on upper positions, and the first notches 41u form a structure that allows easily curving (bending) the first tubular portion 51 forming the first bending portion 22 in the upward direction.

The first notches 41d are also formed in the circumferential direction of the circular tube surface, with a length equal to the length 11u. The first notches 41d are provided horizontally symmetrically in the circumferential direction on left and right sides based on lower positions, and the first notches 41 d form a structure that allows easily curving (bending) the tube body 30 in the downward direction.

Each of the first notches 41 u and 41d is formed in a belt shape in a circumferential direction orthogonal to the longitudinal direction of the circular tube surface (the second notches described later are also formed in the same way).

In the example shown in Figs. 2 and 3, the first notches 41u provided in the upward direction and the first notches 41d provided in the downward direction are provided symmetrically in the vertical direction, and only the positions provided in the longitudinal direction of the tube body 30 are different.

More specifically, the first notch 41d is provided at an intermediate position of two first notches 41u and 41u adjacent in the longitudinal direction of the tube body 30, and the first notch 41u is provided at an intermediate position of two first notches 41d and 41d adjacent in the longitudinal direction.

As shown in Figs. 2 and 5, pulling wires 45u and 45d for actively bending the first bending portion 22 by pulling operation are inserted along an inner wall surface at upper and lower positions inside of the tube body 30, respectively, and distal ends of the pulling wires 45u and 45d are fixed to the inside of the distal end portion of the tube body 30. The pulling wires 45u and 45d are inserted into coil pipes 46u and 46d (so as to form little gaps), in which parts excluding the distal end side are flexible. The pulling operation of the pulling wires 45u and 45d advances and retracts the pulling wires 45u and 45d in the coil pipes 46u and 46d in the longitudinal direction of the tube body 30.

The coil pipes 46u and 46d are formed by closely wound coils (coils formed in a solenoid shape in a degree that adjacent coil parts come into contact), and the coil pipes 46u and 46d are sufficiently flexible even when the coil pipes 46u and 46d are curved. The coil pipes 46u and 46d are also incompressible such that the length hardly changes when the pulling wires 45u and 45d inserted inside advance and retract (or move forward and backward) in the longitudinal direction by the pulling operation.

Distal ends of the coil pipes 46u and 46d are fixed (joined) to the inner wall surface of the tube body 30 by fixing portions (or joint portions) 47, such as soldering, near a front end (distal end) of the second bending portion 23 (second tubular portion 52).

The pulling wires 45u and 45d are exposed from the distal ends of the coil pipes 46u and 46d near the front end of the second bending portion 23 (second tubular portion 52). That is, the pulling wires 45u and 45d exited from the coil pipes 46u and 46d are arranged inside of the first bending portion 22 (first tubular portion 51), and the pulling operation can bend the first bending portion 22. Back ends of the coil pipes 46u and 46d are fixed, for example, inside of the operation portion 12 (not shown). Back ends of the pulling wires 45u and 45d extended toward the back side in the operation portion 12 from the back ends of the coil pipes 46u and 46d are wound around a pulley not shown.

When the pulley rotates in one direction, the back end side of the pulling wire 45u is wound on the pulley, and the pulling wire 45u moves to the back side. When the pulley rotates in the opposite direction, the back end side of the pulling wire 45d is wound on the pulley, and the pulling wire 45d moves to the back side. A rotation axis of the pulley is connected to the bending operation knob 25.

Therefore, for example, the user can perform operation of rotating the bending operation knob 25 in the upward direction to pull the distal end side of the pulling wire 45u through the pulley to bend the first bending portion 22 in the upward direction. The user can perform operation of rotating the bending operation knob 25 in the downward direction to pull the distal end side of the pulling wire 45d through the pulley to bend the first bending portion 22 in the downward direction.

As shown in Fig. 2, guide rings (guide circles) 48u and 48d (not shown in Fig. 3 and subsequent drawings) forming guide portions for moving the pulling wires 45u and 45d in the upward direction and the downward direction in the tube body 30 and restricting movement in other directions are provided on the tube body 30 inside of the first bending portion 22. In the guide rings 48u and 48d, for example, slits are provided on the circumference surface at positions where the bending direction becomes upward and downward in the tube body 30, and each slit part is slightly curved inside in an arc shape. The pulling wires 45u and 45d are inserted into the curved parts such that the pulling wires 45u and 45d can freely advance and retract, and the positions where the pulling wires 45u and 45d are inserted are restricted such that the pulling wires 45u and 45d move only along the positions in the upward direction and the downward direction of bending of the tube body 30 when the first bending portion 22 is bent.

In this way, the first tubular portion 51 of the first bending portion 22 is formed.

Second notches 42u and 42d forming the second notch portion 42 are vertically symmetrically formed throughout a predetermined length from the back end of the first bending portion 22 in the tube body 30 (in the longitudinal direction of the tube body 30), in the longitudinal direction of the circular tube surface, at intervals of, for example, d2a, and with a notch width w2a (in the longitudinal direction of the circular tube surface).

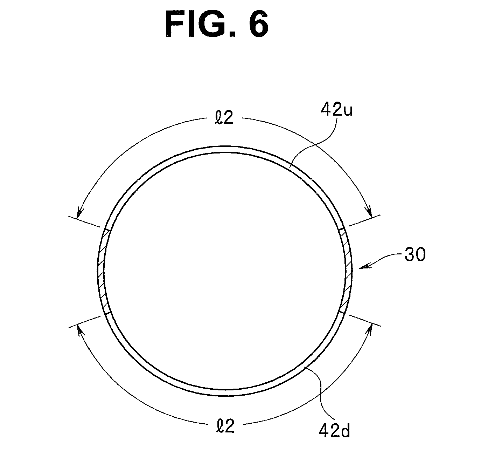

The second notches 42u are formed at a length 12 slightly shorter than half the length of the circular tube surface in the circumferential direction as shown in Fig. 6. The second notches 42u are horizontally symmetrically provided in the circumferential direction on left and right sides based on the position where the bending direction becomes upward, and the second notches 42u form a structure that allows easily curving (bending) the second tubular portion 52 in the upward direction.

The second notches 42d are also formed in the circumferential direction of the circular tube surface, at a length equal to the length 12. The second notches 42d are provided horizontally symmetrically in the circumferential direction on horizontal direction sides based on positions where the bending direction becomes downward, and the second notches 42d form a structure that allows easily curving (bending) the second tubular portion 52 in the downward direction.

Note that the intervals (pitch) d2a in the longitudinal direction of the tube body 30 of adjacent second notches 42u and 42d forming the second tubular portion 52 have a value greater than the intervals (pitch) d1a in the longitudinal direction of the tube body 30 of adj acent first notches 41 u and 41d forming the first tubular portion 51. Therefore, a bending rigidity of the first tubular portion 51 forming the first bending portion 22 when the first tubular portion 51 is curved in the upward or downward direction as a bending direction in which the first bending portion 22 can be actively bent is smaller than a bending rigidity of the second second tubular portion 52, and the first tubular portion 51 can be more easily bent. That is, the bending rigidity of the second bending portion 23 is set to be greater (more difficult to curve) than the bending rigidity of the first bending portion 22, with respect to the bending rigidity in the bending direction in which the first bending portion 22 is bendable.

In the present embodiment, notches 421 and 42r are further provided, for example, horizontally symmetrically on a cylindrical surface facing the horizontal direction in the tube body 30 to form the second notch portion 42 (note that only the notches 421 are illustrated in Figs. 2 and 3, and the other notches 42r are provided at positions overlapping the notches 421 on the lower side of the paper surface in Fig. 2).

The notches 421 and 42r are formed at the same intervals d2a as the second notches 42u and 42d in the longitudinal direction of the tube body 30. The notches 421 and 42r are formed such that a notch width in the longitudinal direction of the tube body 30 is w2b, and a length in the circumferential direction is, for example, 12b. Note that the length 12b shown in Fig. 3 is actually a length along the curved surface.

In this way, the respective notches 421 and 42r are also provided on the cylindrical surface facing the horizontal direction in addition to the cylindrical surface facing the vertical direction in the tube body 30 in the present embodiment to thereby form a structure that allows the second tubular portion 52 forming the second bending portion 23 to easily curve (bend) in the horizontal direction in addition to the vertical direction. As described later, excellent insertion of the insertion portion 11 into a three-dimensionally and intricately curved conduit can be secured.

Note that each of the second notches 42u, 42d, 421, and 42r is also formed in a belt shape in the circumferential direction (on the cylindrical surface) orthogonal to the longitudinal direction of the tube body 30. In this way, the second tubular portion 52 constituting the second bending portion 23 is formed.

Note that as described above, the distal ends of the coil pipes 46u and 46d, into which the pulling wires 45u and 45d are inserted to be able to freely advance and retract, are fixed (joined) near the distal end of the second tubular portion 52 forming the second bending portion 23.

Therefore, when the pulling wires 45u and 45d are pulled, only the first bending portion 22, in which the pulling wires 45u and 45d exposed from the coil pipes 46u and 46d are arranged inside, is bent in the upward or downward direction. In this case, the second bending portion 23 is hardly affected by the bending of the first bending portion 22, and the second bending portion 23 functions to bend (curve) along the curved conduit in the body cavity.

Furthermore, the third notch portion 43 is formed by providing third notches 43a constituting the third notch portion 43 provided throughout the length from the back end of the second bending portion 23 in the tube body 30 to the proximal end of the insertion portion 11, in the longitudinal direction of the circular tube surface, and at intervals of d3a for example, wherein a notch width w3a is provided in a spiral shape. Note that the interval d3a is greater than the interval d2a, for example.

In this way, the flexible tube portion 24 with flexibility is formed by the third tubular portion 53 provided with the third notch portion 43.

The intervals d3a of the third notches 43a in the longitudinal direction are constant at any position in the circumferential direction, and the third tubular portion 53 curves at an equal bending rigidity in any direction. The bending rigidity (value of the bending rigidity) of the flexible tube portion 24 in the bending direction (vertical direction in the present embodiment) in which the first bending portion 22 is bendable is equal to or greater than the bending rigidity of the second bending portion 23 in the present embodiment.

In this way, the endoscope 2 of the present embodiment includes: the long insertion portion 11 inserted into a subject and sequentially provided with the first bending portion 22, the second bending portion 23, and the flexible tube portion 24 from a distal end side; the first tubular portion 51 provided inside of the first bending portion 22, wherein the first notch portion 41 defining a bending direction is provided in a longitudinal direction, the pulling wires 45u and 45d are fixed to the distal end portion 21 of the insertion portion 11, and the pulling wires 45u and 45d are disposed inside; the second tubular portion 52 provided inside of the second bending portion 23, wherein the second notch portion 42 in a pattern different from the first notch portion 41 is provided in a longitudinal direction; and the third tubular portion 53 provided inside of the flexible tube portion 24, wherein the third notch portion 43 in a pattern different from the first and second notch portions 41 and 42 is provided in a longitudinal direction different from the direction in which the second notch portion 42 is provided, wherein the first to third tubular portions 51, 52, and 53 are formed in the tube body 30 as one tubular member, the first notch portion 41 is formed by the first notches 41 u and 41 d bendable at least in two directions, the second notch portion 42 includes the second notches 421 and 42r formed in the two directions as well as the second notches 421 and 42r formed in a direction different from the two directions, and distal end portions of the coil pipes 46u and 46d into which the pulling wires 45u and 45d are inserted to be able to freely advance and retract inside of the second tubular portion 52 are fixed to the second tubular portion 52.

Note that reference signs are provided in the expression described above, and "the second notch portion 42 includes the second notches 421 and 42r formed in the two directions as well as the second notches 421 and 42r formed in a direction different from the two directions" is clear. However, the expression tends to be unclear without reference signs. Therefore, it can be expressed that "the second notch portion includes second notches formed in two directions as well as third notches formed in a direction different from the two directions".

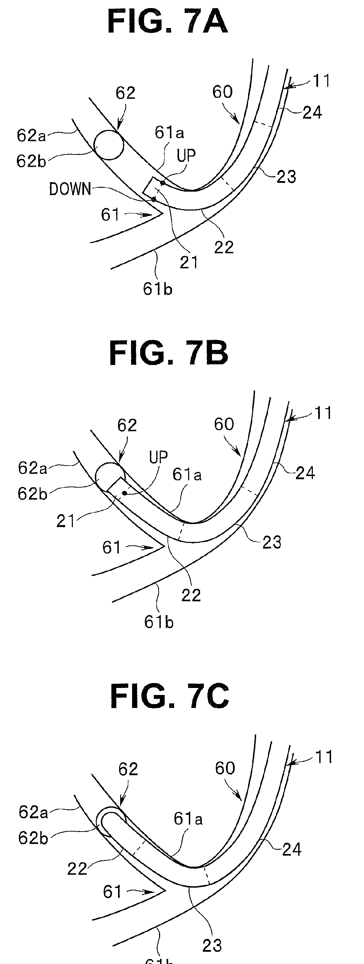

Next, action of the present embodiment will be described with reference to Figs. 7A, 7B, and 7C. Figs. 7A to 7C show part of a conduit 60 (in a subject), such as bronchus, and show a state in which the distal end side of the insertion portion 11 is sequentially inserted toward a deeper side in the conduit 60.

As shown in Fig. 7A, the conduit 60 is branched into a first branch conduit 61 a and a second branch conduit 61b at a first branch portion 61 in the middle. For example, when the distal end of the insertion portion 11 is to be inserted toward the first branch conduit 61 a, the direction of curving from the conduit 60 toward the first branch conduit 61 a can be set by setting the bending direction in the first bending portion 22 to upward or downward. The bending operation knob 25 can be rotated and operated to bend the first bending portion 22 toward the first branch conduit 61 a, and the insertion portion 11 can be pushed and inserted. In this way, the distal end side of the insertion portion 11 can be inserted into the first branch conduit 61a as shown in Fig. 7A.

In Fig. 7A, an orientation that the bending direction in the circumferential direction in the first bending portion 22 is upward and an orientation that the bending direction is downward are indicated by points. Note that upward and downward may be switched to insert the distal end side of the insertion portion 11.

The first branch conduit 61 a is branched into a first branch conduit 62a and a second branch conduit 62b at a second branch portion 62 that is a position on a deeper side of the first branch conduit 61 a. Although the first branch conduit 62a extends in an extension direction of the conduit of the first branch conduit 61 a, the second branch conduit 62b extends in a direction perpendicular to a plane including the first branch portion 61. In the example of Fig. 7A, the second branch conduit 62b extends toward a lower side perpendicular to the paper surface.

When it is desired to insert the distal end side of the insertion portion 11 into the second branch conduit 62b, the insertion portion 11 is rotated 90 degrees in the circumferential direction (by rotation operation of the rotation operation portion 26) from the state of the bending direction of the first bending portion 22 shown in Fig. 7A, and the upward direction or the downward direction of bending in the first bending portion 22 is brought into line with the direction of curving of the second branch conduit 62b from the first branch conduit 61 a.

Fig. 7B shows a state in which the bending direction of the first bending portion 23 is adjusted 90 degrees to bring the orientation in the downward direction of the bending of the first bending portion 22 into line with the direction of curving of the second branch conduit 62b, for example. The user can perform operation of bending the first bending portion 22 in a direction of the extension of the second branch conduit 62b (bent in the downward direction in Fig. 7B) and perform operation of pushing and inserting the insertion portion 11 to thereby insert the distal end side of the insertion portion 11 into the second branch conduit 62b as shown in Fig. 7C.

In the present embodiment, even in the insertion into a conduit such as the second branch portion 62 branching in, for example, the direction orthogonal to the surface of branching of the first branch portion 61 at a short distance from the part of branching in the first branch portion 61 as shown in Fig. 7A to 7C, smooth insertion is possible as in the action described above, because the second bending portion 23 includes the notches 421 and 42r as easily bendable notch portions provided in the bending direction (vertical direction) in which the first bending portion 22 is bendable as well as in a direction different from the bending direction (such as orthogonal horizontal direction).

According to the present embodiment, excellent insertion into a three-dimensionally and intricately curved conduit can be secured. The user, such as an operator, can perform an endoscopic examination or the like in a short time.

On the other hand, if the notches 421 and 42r are not provided, it is difficult to smoothly perform the operation (work) of insertion. For example, in a transition from Fig. 7A to Fig. 7B, the second bending portion 23 needs to be easily bent in the horizontal direction (when the second bending portion 23 is to be inserted toward the deeper side of the first branch conduit 61 a), after the second bending portion 23 is rotated 90 degrees from the state that the second bending portion 23 can be easily bent in the vertical direction. However, the condition cannot be satisfied if the notches 421 and 42r are not provided, and the work of insertion becomes difficult.

According to the present embodiment, a single tubular member forms the first bending portion 22 and the second bending portion 23. This can prevent generation of a difference in level between the first bending portion 22 and the second bending portion 23, and the diameter can be reduced. Furthermore, when the pulling wires 45u and 45d are pulled to bend the first bending portion 22, bending of the second bending portion 23 in the same direction as the first bending portion 22 can be prevented, because the distal ends of the coil pipes 46u and 46d are fixed to the second bending portion 23. That is, there is an advantageous effect that when the first bending portion 22 is bent, a reduction (deterioration) of the insertion due to the bending of the second bending portion 23 in the same direction as the first bending portion 22 as in the conventional example can be eliminated.

The present embodiment is not limited to the specific example described above, and the present embodiment may have structures as illustrated in modifications as shown in Figs. 8 to 17. Note that the following Figs. 8 to 17 illustrate structures of notch portions provided in the tube body 30. The configuration of the inside and the outside of the tube body 30 is the same as in the first embodiment. A regularity of at least one pattern of the pattern (notch pattern) of the first notch portion 41, the pattern (notch pattern) of the second notch portion 42, and the pattern (notch pattern) of the third notch portion 43 changes in the longitudinal direction of the tube body 30 (insertion portion 11). As for the regularity of the pattern, the intervals (pitch) between adjacent notches of a plurality of notches provided in the longitudinal direction of the tube body 30 and the notch width of each of the plurality of notches change in the longitudinal direction of the tube body 30 (insertion portion 11), as can be understood from the modifications described below.

In Fig. 8 of a first modification, the pattern of the first notches provided in the first tubular portion 51 forming the first bending portion 22 is not constant in the longitudinal direction of the tube body 30 and changes in the middle.

In the example illustrated in Fig. 8, the first notch portion 41 includes the first notches 41u and 41d on a front side in the first bending portion 22 of the tube body 30 and first notches 41u' and 41d' on a back side. The first notches 41u and 41d on the front side are formed, for example, in the same way as the first notches 41 u and 41d of Fig. 3, and the first notches 41u' and 41d' on the back side are provided throughout a proper length on the tube body 30 part on the back side, from a position where the first notches 41 u and 41d are provided for a predetermined length.

The first notches 41u' and 41d' on the back side are formed such that intervals d1a' in the longitudinal direction of the tube body 30 are greater than the intervals d1a of the first notches 41 u and 41 d on the front side. The second notch portion 42 forming the second tubular portion 52 and the third notch portion 43 forming the third tubular portion 53 have the same configurations as in the first embodiment.

Although at least a distal end side part (or front side part) of the first bending portion 22 provided on the distal end side of the insertion portion 11 needs to have a structure that can be easily curved (bent), a back side part does not have to be as flexible (bendable) as the distal end side part in many cases. According to the structure as in Fig. 8, the first notches 41 u and 41 d on the front side can be bent to a greater extent (instead, the amount of bending of the first notches 41 u' and 41 d' on the back side is suppressed) when the pulling wire 45u or 45d is pulled. In other words, there is an advantageous effect that the insertion into a greatly curved conduit is facilitated (for a short distance). In addition, there are the same advantageous effects as in the first embodiment.

Although the regularity is changed by making the intervals d1a' of the first notches 41u' and 41d' provided in the longitudinal direction of the tube body 30 different from the intervals d1a of the first notches 41u and 41d in Fig. 8, the width (that is, notch width) of the first notches 41u' and 41d' on the back side may be changed as in a second modification shown in Fig. 9. In the example illustrated in Fig. 9, a notch width w2a' of the first notches 41u' and 41d' on the back side is, for example, greater than the notch width w1a of the first notches 41 u and 41 d on the front side. Note that although the intervals of providing the first notches 41u' and 41d' on the back side (as shown in Fig. 9) in the longitudinal direction of the tube body 30 are also changed (intervals are increased in Fig. 9), the intervals may not be changed.

The second notch portion 42 forming the second tubular portion 52 and the third notch portion 43 forming the third tubular portion 53 have the same configurations as in the first embodiment. In the case of Fig. 9, the same advantageous effects as in the case of Fig. 8 can be obtained, or advantageous effects close to the first embodiment can be obtained, depending on the size of the intervals and the size of the width of the notches.

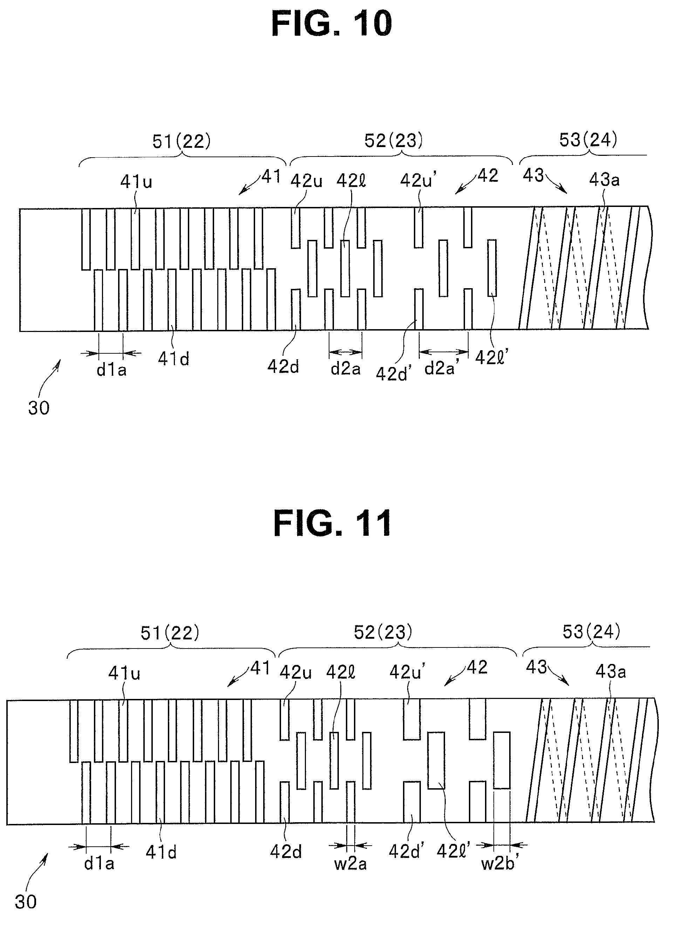

In the structure described in Figs. 8 and 9, the pattern of the first notches provided in the first tubular portion 51 forming the first bending portion 22 in the first embodiment is not constant in the longitudinal direction of the tube body 30 and changes in the middle. However, the pattern of the second notches provided in the second tubular portion 52 forming the second bending portion 23 may not be constant in the longitudinal direction of the tube body 30 and may change in the middle as shown in Figs. 10 and 11.

In the example illustrated in Fig. 10, the second notches 42u, 42d, 421, and 42r on the front side and second notches 42u', 42d', 42l', and 42r' on the back side are included. The second notches 42u, 42d, 421, and 42r on the front side are formed, for example, in the same way as the second notches 42u, 42d, 421, and 42r of Fig. 3, and the second notches 42u', 42d', 42l', and 42r' on the back side are formed such that intervals d2a' in the longitudinal direction of the tube body 30 are greater than the intervals d2a of the second notches 42u, 42d, 421, and 42r on the front side.

The first notch portion 41 forming the first tubular portion 51 and the third notch portion 43 forming the third tubular portion 53 have the same configurations as in the first embodiment. It is often desirable that the front side part of the second bending portion 23 can be more easily curved (bent) than the back side part. Therefore, the configuration of Fig. 10 conforms to such a case.

Although the intervals of providing the second notches 42u and 42d in the longitudinal direction of the tube body 30 are changed in Fig. 10, a notch width of the second notches 42u', 42d', 42l', and 42r' on the back side may be changed as in a fourth modification shown in Fig. 11.

In the example illustrated in Fig. 11, a notch width w2b' of the second notches 42u', 42d', 42l', and 42r' on the back side is greater than the notch width w2a of the second notches 42u, 42d, 421, and 42r on the front side. Note that the intervals of providing the second notches 42u', 42d', 42l', and 42r' on the back side in the longitudinal direction of the tube body 30 may also be changed (as shown in Fig. 11), or the intervals may not be changed. The first notch portion 41 forming the first tubular portion 51 and the third notch portion 43 forming the third tubular portion 53 have the same configurations as in the first embodiment.

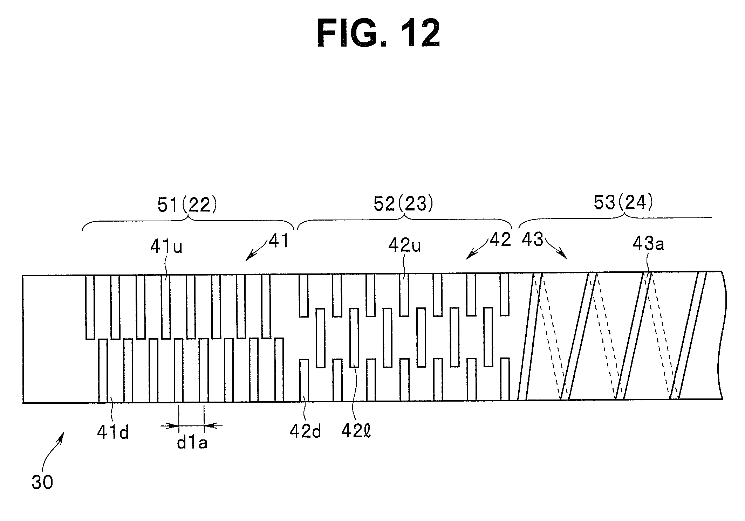

As in a fifth modification shown in Fig. 12, the pattern of the third notches forming the third notch portion 43 provided in the third tubular portion 53 may not be constant in the longitudinal direction of the tube body 30 and may change in the middle.

In the example shown in Fig. 12, the intervals (pitch) of the third notches in the longitudinal direction of the tube body 30 increase toward the back side. Note that the intervals may be constant near the back side. The first tubular portion 51 (first bending portion 22) and the second tubular portion 52 (second bending portion 23) have, for example, the same configurations as in the first embodiment.

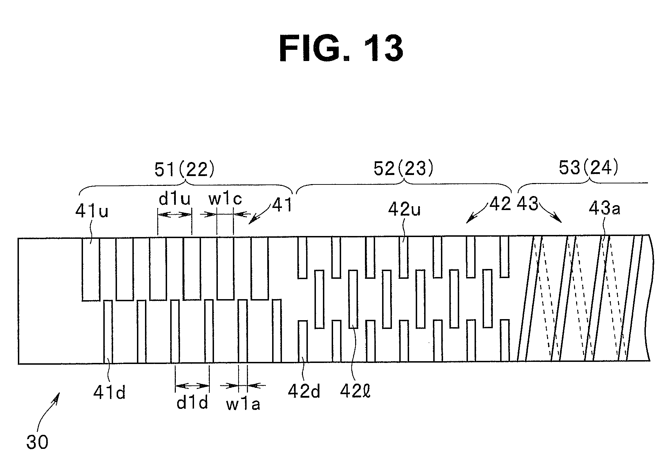

Fig. 13 shows a structure of notch portions provided in the tube body 30 in a sixth modification. In the first embodiment, the first notches 41u and 41 d forming the first notch portion 41 constituting the first tubular portion 51 are substantially the same notches, except that the positions in the longitudinal direction are shifted. In the present modification, a notch width w1c of the first notches 41 u for bending in the upward direction is greater than the notch width w1a of the first notches 41 d for bending in the downward direction, for example.

Note that the intervals (pitch) d1u and d1d of the first notches 41u and the first notches 41d in the longitudinal direction of the tube body 30 have the same values. According to the structure, the amount of bending in the upward direction can be greater than the amount of bending in the downward direction when the pulling wire 45u or 45d is pulled. Therefore, bending in the upward direction can be selected and used when the bending has to be large, and bending in the downward direction can be selected and used when the bending does not have to be large. The second tubular portion 52 (second bending portion 23) and the third tubular portion 53 (flexible tube portion 24) are the same as in the first embodiment.

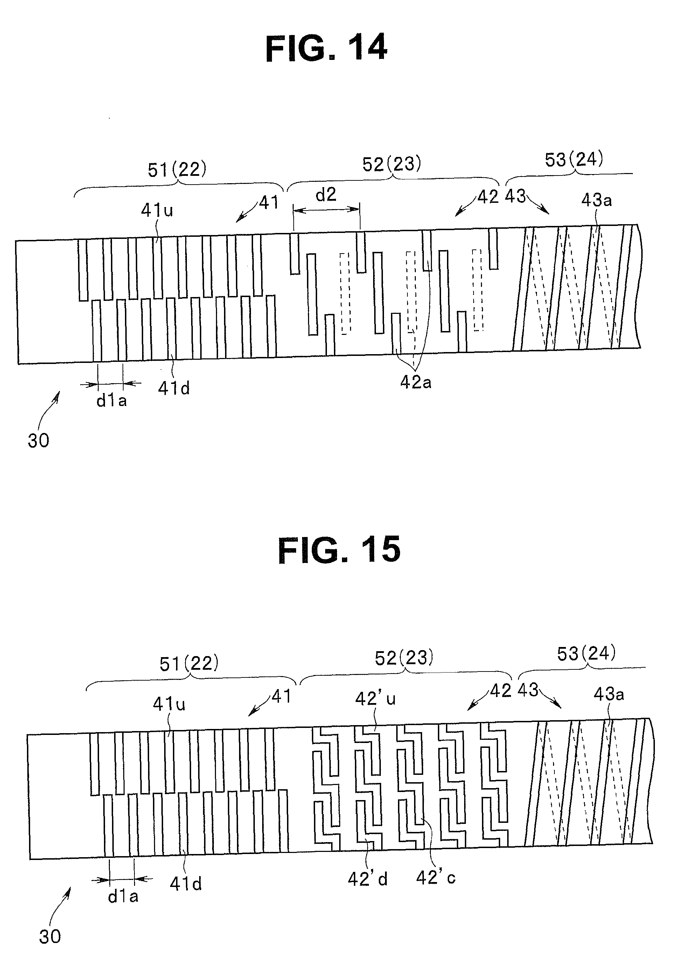

Fig. 14 shows a structure of notch portions provided in the tube body 30 according to a seventh modification of the first embodiment. In the present modification, the structure of the second notch portion 42 provided in the second tubular portion 52 forming the second bending portion 23 is changed in the first embodiment, for example.

The second notch portion 42 according to the present modification includes second notches 42a formed along the spiral on the cylindrical surface of the tube body 30. Although the third notches 43a in the first embodiment are continuously formed along the spiral, the second notches 42a are discretely and periodically formed along the spiral in the present modification. Fig. 14 shows an example in which the pitch of the spiral is d2. In the example shown in Fig. 14, the notches 42a are periodically formed in the bending direction (vertical direction) in which the first bending portion 22 is bendable, and bending (curving) in the direction is facilitated. Furthermore, the notches 42a are periodically formed such that bending (curving) in other directions is also facilitated.

The first notch portion 41, the third notch portion 43, and the like other than the second notch portion 42 have the same configurations as in the first embodiment, for example. In the second bending portion 23 of the present modification, bending (curving) in the bending direction in which the first bending portion 22 is bendable is facilitated, and bending (curving) in other directions is also facilitated. Therefore, the present modification also has the advantageous effects included in the first embodiment.

Fig. 15 shows a structure of notch portions provided in the tube body 30 according to an eighth modification of the first embodiment. In the present modification, the structure of the second notch portion provided in the second tubular portion 52 forming the second bending portion 23 is changed in the first embodiment, for example.

The second notch portion 42 in the present modification is provided with hook-shaped notches 42'u and 42'd at positions in the upward direction of bending and positions in the downward direction of bending in the longitudinal direction of the tube body 30, respectively, and is also provided with hook-shaped notches 42'c in the horizontal direction of bending. Note that the hook-shaped notches 42'c are also horizontally symmetrically provided on a surface that is the back side of the paper surface.

Similarly, in the present modification, the second bending portion 23 can be easily bent (curved) in the bending direction in which the first bending portion 22 is bendable due to the hook-shaped notches 42'u and 42'd and can also be bent (curved) in other directions due to the hook-shaped notch 42'c. Therefore, the present modification also has the advantageous effects included in the first embodiment.

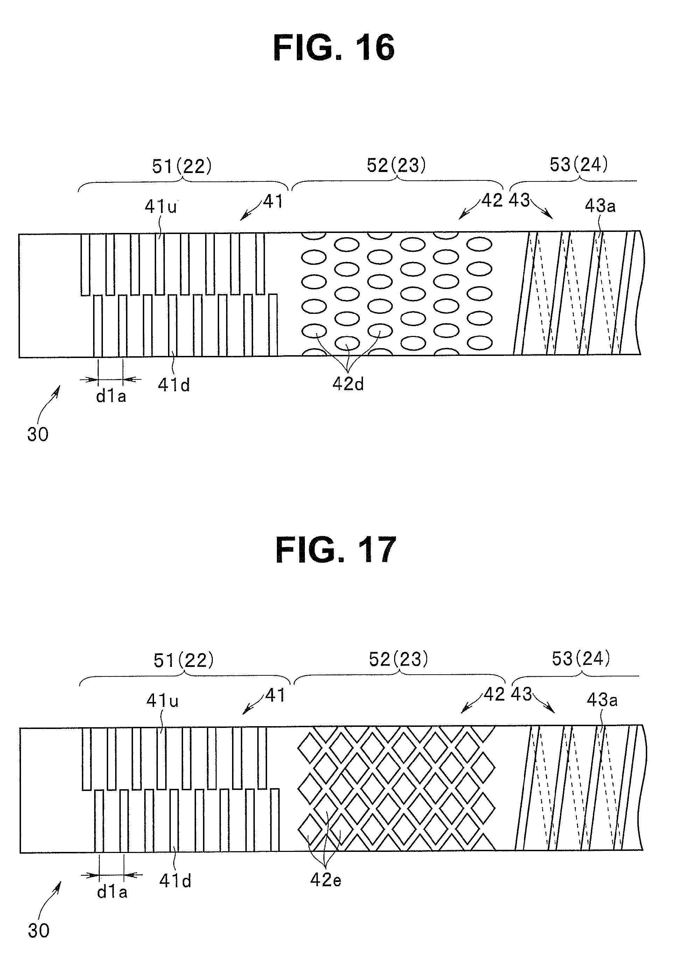

Fig. 16 shows a structure of notch portions provided in the tube body 30 according to a ninth modification of the first embodiment. In the present modification, the structure of the second notch portion 42 provided in the second tubular portion 52 forming the second bending portion 23 is changed in the first embodiment, for example. More specifically, for example, elliptical notches (or holes) 42d as the second notch portion 42 are periodically formed in the longitudinal direction and the circumferential direction of the tube body 30.

Note that although the elliptical notches (or holes) 42d are provided in the example illustrated in the present modification, rhombic notches (or holes) 42e may be periodically formed in the longitudinal direction and the circumferential direction of the tube body 30 as in a tenth modification shown in Fig. 17. Other than the rhombic notches 42e, the notches may be formed in a hexagonal or octagonal shape, for example.

Embodiments and the like formed by partially combining the embodiment and the modifications described above also belong to the present invention. For example, although Figs. 10 and 11 illustrate configurations in which the second tubular portion 52 in the first embodiment (Fig. 3) is modified, the configuration may also be applied to the modification of Fig. 8 or 9.

Although Fig. 12 illustrates a configuration in which the third tubular portion 53 in the first embodiment (Fig. 3) is modified, the configuration may also be applied to one of the modifications in Figs. 8 to 11. Although Fig. 13 illustrates a configuration in which the first tubular portion 51 in the first embodiment (Fig. 3) is modified, the configuration may also be applied to one of the modifications in Figs. 8 and 10 to 12. The embodiment to be applied may also be changed in Figs. 14 to 17 and the like.

The present application is filed on the basis of claiming the benefit of priority from Japanese Patent Application No. 2014-079765, filed on April 8, 2014 in Japan, and the disclosed contents are incorporated in the present specification and the claims by reference.

## Claims

1. An endoscope comprising:
a long insertion portion inserted into a subject and sequentially provided with a first bending portion, a second bending portion, and a flexible tube portion, from a distal end side;
a first tubular portion provided inside of the first bending portion, wherein a first notch portion defining a bending direction is provided in a longitudinal direction, pulling wires are fixed to a distal end portion of the insertion portion, and the pulling wires are disposed inside;
a second tubular portion provided inside of the second bending portion, wherein a second notch portion in a pattern different from a pattern of the first notch portion is provided in a longitudinal direction; and
a third tubular portion provided inside of the flexible tube portion, wherein a third notch portion in a pattern different from the patterns of the first and second notch portions is provided in a longitudinal direction different from the direction in which the second notch portion is provided, wherein
the first to third tubular portions are formed in one tubular member,
the first notch portion is formed of first notches bendable at least in two directions,
the second notch portion includes second notches formed in the two directions and third notches formed in a direction different from the two directions, and
in the second tubular portion, distal end portions of coil pipes into which the pulling wires are inserted to be able to freely advance and retract are fixed to inside of the second tubular portion.

2. The endoscope according to claim 1, wherein
the first tubular portion comprises guide portions configured to guide the pulling wires such that the pulling wires can freely advance and retract.

3. The endoscope according to claim 1, wherein
the third notch portion includes notches continuous in a spiral shape.

4. The endoscope according to claim 1, wherein
a mesh tube is disposed outside of a member of the one tubular portion, a tubular elastic member is further disposed outside of the mesh tube outside of parts equivalent to the first and second notch portions, and a tubular resin member is disposed outside of the mesh tube outside of a part equivalent to the third notch portion.

5. The endoscope according to claim 1, comprising
the first bending portion, the second bending portion, and the flexible tube portion, wherein a bending rigidity of the second bending portion is greater than a bending rigidity of the first bending portion with respect to a bending rigidity in a bending direction in which the first bending portion is bendable, and a bending rigidity of the flexible tube portion is equal to or greater than the bending rigidity of the second bending portion.

6. The endoscope according to claim 1, wherein
the one tubular portion member is formed of a shape memory alloy.

7. The endoscope according to claim 6, wherein
the shape memory alloy is a nickel-titanium alloy (NiTi).

8. The endoscope according to claim 6, wherein
at least one pattern of the pattern of the first notch portion, the pattern of the second notch portion, and the pattern of the third notch portion is formed such that a regularity of the pattern changes in a longitudinal direction of the insertion portion.

9. The endoscope according to claim 6, wherein
at least one pattern of the pattern of the first notch portion, the pattern of the second notch portion, and the pattern of the third notch portion is formed such that intervals of formation of adjacent notches in a longitudinal direction of the insertion portion or a regularity of a notch width in the longitudinal direction of the insertion portion changes.

10. The endoscope according to claim 1, wherein
the distal end portions of the coil pipes are fixed to an inner surface near a distal end portion of the second tubular portion forming the second bending portion.

11. The endoscope according to claim 1, wherein
the first tubular portion comprises the first notch portion including the plurality of first notches, each provided at a first interval in the longitudinal direction facing first and second directions respectively opposing in a circumferential direction of the first tubular portion, and
the second tubular portion comprises the second notch portion including:
the plurality of second notches, each provided at a second interval in the longitudinal direction facing the first and second directions respectively opposing in a circumferential direction of the second tubular portion; and
the plurality of third notches, each provided at a third interval in the longitudinal direction facing third and fourth directions respectively substantially orthogonal to the first and second directions in the circumferential direction of the second tubular portion.

12. The endoscope according to claim 11, wherein
the second interval of the second notches is formed to be greater than the first interval of the first notches.

13. The endoscope according to claim 12, wherein
in the first tubular portion, front a side interval as an interval of the first notches provided at a front side part in the longitudinal direction of the first tubular portion is smaller than a back side interval as an interval of the first notches provided at a back side part in the longitudinal direction of the first tubular portion.

14. The endoscope according to claim 11, wherein
the coil pipes with incompressibility and flexibility are provided in the flexible tube portion and the second bending portion, wherein a length of the coil pipes hardly changes when the pulling wires advance and retract after the pulling wires are inserted to be able to freely advance and retract.
